Europäisches Patentamt

(19) European Patent Office

Office européen des brevets

(11) Publication number: **0 161 003**
**A2**

## (12) EUROPEAN PATENT APPLICATION

(21) Application number: **85105771.1**

(22) Date of filing: **10.05.85**

(51) Int. Cl.⁴: **A 61 B 5/08**

(30) Priority: **11.05.84 US 609574**

(43) Date of publication of application:
**13.11.85 Bulletin 85/46**

(84) Designated Contracting States:
**DE FR GB IT**

(71) Applicant: CHESEBROUGH-POND'S INC.
33 Benedict Place
Greenwich, Conn. 06830(US)

(72) Inventor: Helfer, Joel N.
253 Weatherside Road
Cheshire Connecticut 06410(US)

(74) Representative: Patentanwälte Grünecker, Dr.
Kinkeldey, Dr. Stockmair, Dr. Schumann, Jakob, Dr.
Bezold, Meister, Hilgers, Dr. Meyer-Plath
Maximilianstrasse 58
D-8000 München 22(DE)

(54) A mouthpiece apparatus for a pulmonary function tester.

(57) A mouthpiece apparatus for a pulmonary function tester, e.g., a spirometer, has a housing with a woven fabric diaphragm positioned across a passage through the housing. The woven fabric diaphragm provides a known differential pressure as a function of air flow, which is substantially the same from unit to unit. A port, capable of being connected to a pressure transducer, is in fluid communication with the passage, and the woven fabric resistance is located downstream of the port. The material used for the woven resistance element is one that is resistant to absorption of moisture and is formed as a monofilament.

FIG.1

GRÜNECKER, KINKELDEY, STOCKMAIR & PARTNER

PATENTANW**0161003**
EUROPEAN PATENT ATTORNEYS

A GRUNECKER DIPL ING
DR H KINKELDEY DIPL ING
DR W STOCKMAIR DIPL ING AE E (CALTECH
DR K SCHUMANN DIPL PHYS
P H JAKOB DIPL ING
DR G BEZOLD DIPL CHEM
W MEISTER DIPL ING
H HILGERS DIPL ING
DR H MEYER PLATH DIPL ING
DR M BOTT-BODENHAUSEN DIPL ING
DR U KINKELDEY DIPL BIOL

LICENCIE EN DROIT DE L UNIV DE GENEVE

8000 MÜNCHEN 22
MAXIMILIANSTRASSE 58

# A MOUTHPIECE APPARATUS FOR A PULMONARY FUNCTION TESTER

The invention relates to a mouthpiece apparatus for a pulmonary function tester. More particularly, the invention concerns a mouthpiece apparatus that provides a known pressure differential across a resistance located therein in response to air flowing through the apparatus.

Respiratory flow in patients having respiratory difficulties is measured by a viariety of devices. U.S. Patent No. 4,083,245 to Osborn describes a known device for making such a measurement, i.e., a resistance pneumotachograph. This instrument operates to determine air flow and volume by measuring the pressure differential created by air flowing across a resistance in a tube. The resistance may be provided by an orifice or by many small parallel tubes, as in a Fleisch-type resistance pneumotachograph.

A mouthpiece apparatus for a pulmonary function tester using an oriface as a resistance has a square-law relation of air flow to pressure differential. For clinical evaluation it is desirable that a device be capable of measuring air flow volume in the range of 12 ml./sec. to 12 l./sec., a range of 1000-to-1. Accordingly, using a square law oriface resistance element it became necessary to measure pressure over a range of $10^6$. Devices for linearizing the differential pressure versus flow relationship through an orifice have been suggested. For example, U.S. Patent No. 4,083,245 explains and illustrates one such device. The many small parallel tubes of a Fleisch-type pneumotachograph provide a roughly linear differential pressure versus flow relationship, but the required structure is a large, heavy, expensive instrument that is subject to being plugged by either mucous or moisture in the air.

A design objective for a pulmonary function tester, such as, for example, a spirometer, is that it has a mouthpiece apparatus which provides a known and approximately linear differential pressure in response to air flow. The known differential pressure should be substantially identical from unit to unit. Furthermore, the mouthpiece apparatus should be simply and inexpensively constructed, so that it is disposable, and should be easily connectable to electronic equipment, so that it is readily replaceable. A disposable mouthpiece apparatus is desirable in order to prevent cross contamination problems, i.e., one patient who uses the instrument contaminating another patient who uses the instrument.

Accordingly, a need exists for a mouthpiece apparatus for a pulmonary function tester with approximately linear response, that is easily and inexpensively manufactured and, therefore, is replaceable and disposable.

In accordance with the invention, a mouthpiece apparatus for a pulmonary function tester has a generally cylindrical housing with an inlet end, and an outlet end, one of which is formed as a mouthpiece adapted to be inserted into the mouth of a patient, and an internal passage for air flow between the inlet and the outlet. An air-permeable diaphragm, functioning as a flow resistance element, extends completely across the passage and has a surface area selected so that the diaphragm produces a pressure drop of no more than 7 inches of water at air flow rates up to 12 liters per second. The air-permeable diaphragm is fabricated from a woven, moisture-resistant monofilament fabric that has more than 300 threads per inch. The mouthpiece also has a pressure sampling port, which is capable of being connected to a pressure detector and which is located between the mouthpiece end and the diaphragm.

The woven fabric diaphragm produces a known differential pressure profile in response to air flowing through it. This known differential pressure relationship is substantially the same from mouthpiece to mouthpiece, since the manufacturing techniques for the woven fabric permit tight tolerances. Further, this relationship is approximately linear throughout the flow range in question, thereby, facilitating the use of a relatively inexpensive pressure transduced and linearizing scheme. Consequently, the mouthpiece apparatus may be utilized for a diagnostic tester and may be replaced without change in calibration of the tester. Furthermore, the mouthpiece with the woven fabric resistance is easily and inexpensively manufactured, thereby making the mouthpiece apparatus conveniently disposable.

In a preferred embodiment of the invention, the woven fabric is woven from polyester monofilament, has a mesh count of approximately 471 per inch and a mesh opening of approximately .0008 inch, and provides a pressure differential of approximately 4 inches of water at an air flow of 12 liters/sec. The woven fabric insert may advantageously be held between portions of the housing which are joined.

In a preferred embodiment of the invention, for measuring expiration functions, the housing has two sections. The inlet end mouthpiece and the port are located in the first section, while the outlet end is located in the second section. The woven fabric is mounted between the sections which are ultrasonically sealed together. The inlet end mouthpiece advantageously has a cross-sectional area of approximately 1.23 in.$^2$, and the woven fabric resistance has a cross-sectional area of approximately 3.14 in.$^2$. Preferably, the housing is contoured to permit a smooth transition from the smaller cross section to the larger cross section without creating unnecessarily turbulent air flow.

The above and other advantages and objects of the present invention may be better understood by reference to the following description of an exemplary embodiment thereof taken in conjunction with the accompanying drawings, in which:

Figure 1 is a cross-sectional view of a mouthpiece according to the invention; and

Figure 2 is a front-elevational view with parts broken away for clarity of a mouthpiece according to the invention.

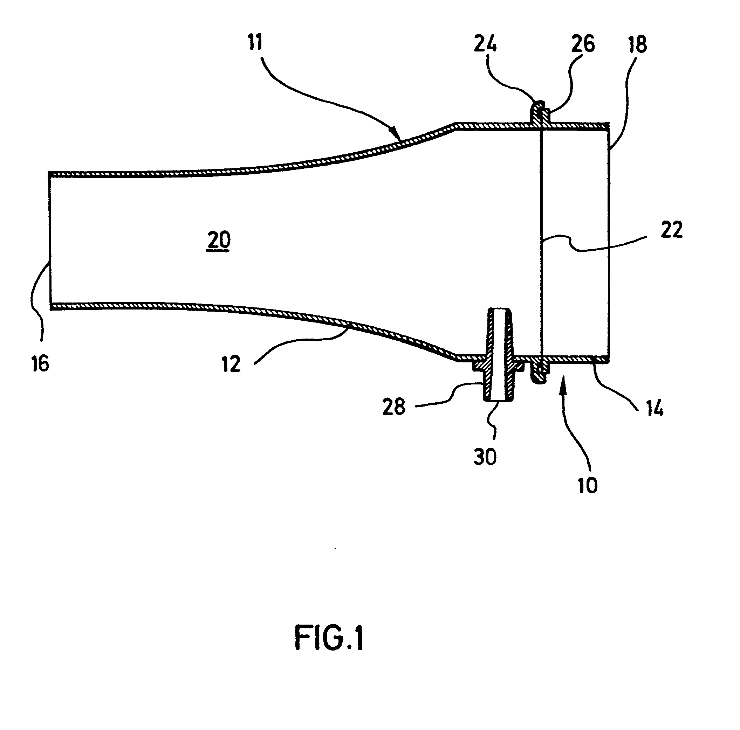

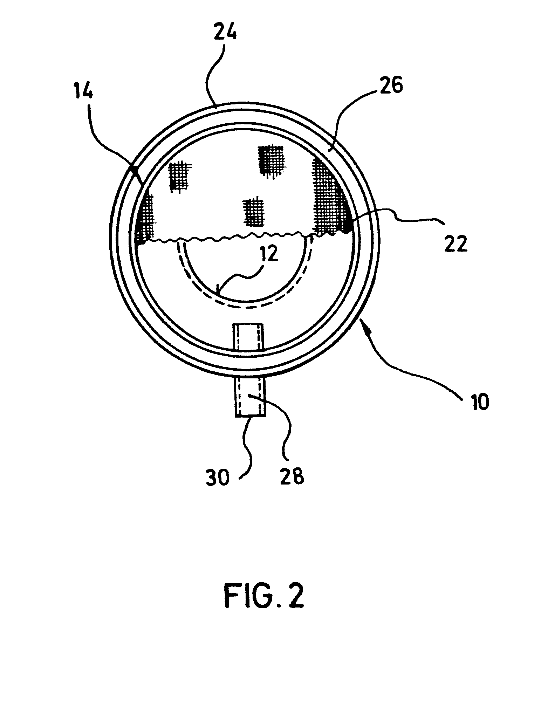

An ex emplary embodiment of a mouthpiece apparatus 10 in accordance with the invention, for use in measuring expiration pulmonary functions, is illustrated in Figures 1 and 2, in which like reference numerals designate like elements. The mouthpiece apparatus 10 has a housing 11 that includes two sections, an upstream section 12 and downstream section 14, each section being molded from plastic material, such as polystyrene. The sections 12 and 14 include flanges 24 and 26, respectively. The mouthpiece apparatus is simply and easily constructed by inserting the flange 26 into a recessed portion of the flange 24 with a woven fabric resistance diaphragm 22 positioned therebetween. The two sections are then joined together using known techniques, such as adhesive or ultrasonic bonding.  The woven fabric diaphragm 22 is advantageously held by ultrasonic sealing of the housing sections.  A passage 20 within the housing 11 connects an inlet end 16 which forms a mouthpiece with an outlet end 18.

A tube 28 in the housing defines a port 30 that may be connected to a pressure transducer (not shown) via a hose (not shown).  The pressure transducer supplies a signal indicative of the pressure difference generated by the air flowing through the mouthpiece to electronic circuitry, which may provide calibration correction and which calculates various parameters concerning the respiratory or pulmonary capacity of a patient flowing through the mouthpiece apparatus, such as forced vital capacity (FVC), forced expiratory volume in one second ($FEV_1$), $FEV_1$/FVC ratio, peak flow, forced expiratory flow 25% to 75% and extrapolated volume.

The upstream section 12 of the housing 11 is tapered to provide a smooth transition from a smaller to a larger diameter without creating excessively turbulent air flow. The diameter of the passage at the inlet end is 1.25 in. (corresponding to a cross-sectional area of 1.23 $in.^2$), which is common, because it allows the mouthpiece end to fit into the mouth of the patient without permitting the patient to purse his lips.  The passage gradually widens when moving from the inlet 16 to the outlet 18 (i.e., in the downstream direction) and has a diameter of 2.0 in. (corresponding to a cross-sectional area of 3.14 $in^2$) at the position of the woven fabric resistance diaphragm 22.

The cross-sectional area at the location of the air permeable woven fabric diaphragm 22 and the parameters of the diaphragm are selected so that the differential pressure across the resistance does not impede the air flow greatly or cause an excessive back pressure.  An excessive back pressure is undesirable because it may impair the breathing of the patient. A woven fabric resistance that provides a differential pressure of about 4 inches of water at an air flow of approximately 12 liters/sec is an example of one such woven fabric resistance. More specifically, a woven polyester monofilament insert with the following specifications that is placed across a 3.14 in.$^2$ flow area provides suitable pressure differentials at typical air flow rates:

| | |
|---|---|
| mesh opening | .0008 inch |
| mesh count | 470.8 per in. |
| thread diameter | .0013 inch |
| open area | 15% |
| fabric thickness | .0028 inch |
| weight | 1.47 oz. per sq. yd. |
| tensile strength (of a 2 in. by 8 in. strip) | 84/86 lbs. warp/weft and 23/34 elongation % |

Tetko Inc. manufactures a woven polyester monofilament fabric with the above specifications, which is available under model number HD 7-21.

Polyester monofilament is preferred because it absorbs very little moisture - which might alter the differential pressure profile - and it is easily formed as a monofilament, i.e., a single, untwisted strand. A fabric woven from a monofilament is desirable because moisture cannot be trapped between the fibers of the threads (since, by definition, each thread is a single strand). However, monofilament materials other than polyester may be used to fabricate the woven resistance element. Polyester has a water absorption of 0.3%. Nylon, with a slightly higher absorption of 3% could also be used.

While the invention is described with respect to a device for measuring expiration functions, it will be evident to those skilled in the art that the principles of the invention can also be applied to devices for measuring inspiration functions.

Although the invention has been described herein with respect to specific embodiment thereof, it will be understood that various modifications and changes may be made thereto without departing from the inventive concepts disclosed. All such modifications and changes are intended to be included within the spirit and scope of the appended claims.

CLAIMS:

1. A mouthpiece apparatus for a pulmonary function tester, comprising a generally cylindrical housing having a first inlet end and a second outlet end, one of which is formed as a mouthpiece and adapted to be inserted into the mouth of a patient and an internal passage for flow of air between said first and second ends, a flow resistance element in said passage, said flow resistance element comprising an air-permeable diaphram fabricated of a woven, moisture resistant monofilament fabric having in excess of 300 threads per inch, said diaphragm extending completely across said passage and having a surface area selected so that said diaphragm provides a pressure drop of no more than 7 inches of water at an air flow rate of up to 12 liters per second, and a pressure sampling port in said housing between said mouthpiece end and said diaphragm.

2. A mouthpiece apparatus according to claim 1, wherein said surface area of said diaphragm is selected to provide a pressure differential of approximately 4 inches of water at an air flow of 12 liters/sec.

3. A mouthpiece apparatus according to claim 1, wherein the said monofilament is polyester.

4. A mouthpiece apparatus according to claim 2, wherein said woven fabric has a mesh count of approximately 471 per inch and a mesh opening of approximately .0008 inch.

5. A mouthpiece apparatus according to claim 3, wherein said housing is formed in two sections and wherein said woven fabric is held between said section.

6. A mouthpiece apparatus according to claim 4, wherein the first inlet end forms said mouthpiece and has a cross-sectional area of approximately 1.23 in.$^2$ and wherein

said surface area is approximately 3.14 in.$^2$.

7.  A mouthpiece apparatus according to claim 1, wherein the first inlet end forms said mouthpiece has a cross-sectional area of approximately 1.23 in.$^2$ and wherein said surface area is approximately 3.14 in.$^2$.

8.  A mouthpiece apparatus according to claim 1, wherein the housing has a first section and a second section, wherein said first inlet end and said port are in said first section of the housing, wherein said second oulet end is in said second section of the housing, and wherein said diaphragm is located between said first and second sections of the housing.

9.  In an apparatus for measuring the volume of a flow of gas, wherein said gas passes through a flow resistant element in a passage which provides a pressure differential dependent on flow volume, the improvement wherein said flow resistance element comprises a diaphragm extending across said passage and having a fabric woven from monofilament threads of a water-impervious material, said fabric having greater than 300 threads per inch.

0161003

FIG.1

FIG. 2